# EUROPEAN PATENT SPECIFICATION

(11) **EP 3 698 142 B1**
(45) Date of publication and mention of the grant of the patent: **30.04.2025**
(21) Application number: 18789143.7
(22) Date of filing: 19.10.2018
(51) Int. Cl.: G01N 33/68, C07K 16/18, G01N 33/574

(54) **COLLAGEN TYPE XVI ASSAY**
KOLLAGENTYP-XVI-TEST
DOSAGE DE COLLAGÈNE DE TYPE XVI

(30) Priority: 20.10.2017 GB 201717301
(43) Date of publication of application: 26.08.2020
(73) Proprietor: Nordic Bioscience A/S, 2730 Herlev (DK)
(72) Inventor: JENSEN, Christina, DK-2200 Kobenhavn N (DK); MORTENSEN, Joachim, Hog, DK-2670 Hundige Strand (DK); WILLUMSEN, Nicholas, DK-2870 Dyssegard (DK); KARSDAL, Morten, Asser, DK-2100 Kobenhavn (DK)
(74) Representative: Beck Greener LLP
(86) International application number: PCT/EP2018/078697
(87) International publication number: WO 2019/077102

(56) References cited:
- EP-A1- 2 680 003
- US-A1- 2010 240 077
- US-A1- 2016 178 644
- IMMUNOGEN: "PAC136Hu01 Polyclonal Antibody to Collagen Type XVI (COL16) Organism Species: Homo sapiens (Human) [ PRODUCT INFORMATION ]", 1 January 2014 (2014-01-01), XP055539385, Retrieved from the Internet <URL:http://www.cloud-clone.com/manual/Polyclonal-Antibody-to-Collagen-Type-XVI--COL16--PAC136Hu01.pdf> [retrieved on 20190108]
- SABINE RATZINGER ET AL: "Collagen XVI induces formation of focal contacts on intestinal myofibroblasts isolated from the normal and inflamed intestinal tract", MATRIX BIOLOGY, vol. 29, no. 3, 1 April 2010 (2010-04-01), NL, pages 177 - 193, XP055538888, ISSN: 0945-053X, DOI: 10.1016/j.matbio.2009.11.004
- S. N. KEHLET ET AL: "Excessive collagen turnover products are released during colorectal cancer progression and elevated in serum from metastatic colorectal cancer patients", SCIENTIFIC REPORTS, vol. 6, no. 1, 28 July 2016 (2016-07-28), XP055539306, DOI: 10.1038/srep30599
- JOACHIM HØG MORTENSEN ET AL: "Ulcerative colitis, Crohn's disease, and irritable bowel syndrome have different profiles of extracellular matrix turnover, which also reflects disease activity in Crohn's disease", PLOS ONE, vol. 12, no. 10, 13 October 2017 (2017-10-13), pages e0185855, XP055538751, DOI: 10.1371/journal.pone.0185855
- MARTIN F. GRAHAM ET AL: "Collagen content and types in the intestinal strictures of Crohn's disease", GASTROENTEROLOGY, vol. 94, no. 2, 1 February 1988 (1988-02-01), US, pages 257 - 265, XP055539328, ISSN: 0016-5085, DOI: 10.1016/0016-5085(88)90411-8

## Description

### Field of the Invention

The present invention relates to a type XVI collagen assay and its use in evaluating diseases associated with type XVI collagen, in particular colorectal cancer and ulcerative colitis, and for identifying a subgroup of patients with Crohn's disease that have (or are likely to develop) fibrostenotic strictures.

### Introduction

The extracellular matrix (ECM) is a non-cellular component responsible for maintaining tissue architecture. Altered ECM remodelling is a significant part of the pathology of gastrointestinal (GI) disorders such as colorectal cancer (CRC) (1) and ulcerative colitis (UC) (2). An imbalance between ECM formation and degradation in the colon leads to an altered composition of the ECM thereby causing an abnormal tissue function. Elevated deposition of ECM proteins in the tumour microenvironment increase the stiffness of the ECM, which influences cellular functions such as cell proliferation, adhesion, migration and invasion (3,4). It has also become evident that inflammatory responses in the tumour microenvironment affect the ECM remodelling (5,6). Likewise, in UC, the ECM of the intestine is highly affected by chronic inflammation which leads to loss of tissue homeostasis and imbalanced collagen turnover (2,7-9). The chronic inflammation and the continuous turnover of epithelial cells contribute to development of dysplasia which may transform into CRC (10). Biomarkers reflecting this enhanced ECM remodelling may therefore be important to identify patients with disruption in tissue/ECM architecture responsible for development and progression of CRC and UC. Possible biomarkers previously described include MMP degraded biglycan and collagen V(25). Increased levels of collagen V in the fibrotic submucosa of a strictured bowel have been reported (26).

In the intestine, type XVI collagen (hereinafter col-16) is suggested to contribute to stabilization and maintenance of basement membranes, a specialized layer of ECM located beneath the epithelial and endothelial cell layers (11). Col-16 is a fibril-associated collagen with interrupted triple helices (FACITs), and expressed by epithelial cells and subepithelial myofibroblasts. These are localized subjacent to the basement membrane with a pronounced deposition of col-16 into the matrix of the epithelial crypts (11). Studies of skin show that col-16 is localized in the dermal-epidermal junction zone near basement membranes, which suggests that col-16 has an active role in anchoring microfibrils to basement membranes (12,13).

Col-16 interacts with α1β1 and α2β1 integrins and induces the recruitment of these integrins into focal adhesion plaques, which promotes integrin-mediated cell reactions, such as cell spreading and alterations in cell morphology (14,15). The binding of col-16 to integrins stimulates cell-matrix interactions, which it is postulated may induce an invasive phenotype in tumour cells. Interestingly, overexpression of col-16 has been shown to induce cell invasion and a proliferative cellular phenotype in oral squamous cell cancer (OSCC) (16,17). Col-16 is deposited at the basement membrane in normal oral epithelium while it seems to disappear from the basement membrane in tissues from OSCC patients (17). The loss of col-16 from the basement membrane zone in the development of OSCC may induce ECM remodelling and a disruption of the basement membrane that promotes tumour cell infiltration and a progression of disease. In glioblastomas, col-16 is involved in tumour cell adhesion and invasion as well as tumour specific remodelling of the ECM (18,19). Increased expression of col-16 has also been detected in intestinal subepithelial myofibroblasts isolated from inflamed Crohn's disease tissue biopsies (11).

Biomarkers for diagnosing inflammatory bowel disease, including Crohn's disease, including fibrotic or fibrostenotic Crohn's disease, and ulcerative colitis and methods of using such biomarkers are described in US2016/0178644. US2010/240077 describes diagnosing or predicting susceptibility to a clinical subtype of Crohn's disease in a subject having Crohn's disease by determining the presence or absence of IgA anti-I2 antibodies. Polyclonal antibodies to Col16 are commercially available from Cloud-Clone Corp (PAC136Hu01). EP2680003 describes the use of Col10A1 as a serum protein biomarker for colorectal cancer

The inventors have now established a pathological link between col-16 and UC and CRC, and have also developed a method for identifying a subgroup of patients with Crohn's disease that have (or are likely to develop) fibrostenotic strictures.

### Summary of the Invention

Accordingly, in a first aspect the present invention relates to a method of detecting collagen type XVI or fragments thereof in a human biofluid sample, said method comprising:
contacting a biofluid sample obtained from a human patient with a monoclonal antibody specifically reactive with C-terminus amino acid sequence PMKTMKGPFG (SEQ ID NO: 1) of a C-terminus biomarker and detecting binding between the biomarker and the antibody.

The detection is preferably quantitative.

Preferably, the monoclonal antibody is raised against a synthetic peptide having the amino acid sequence PMKTMKGPFG (SEQ ID NO: 1). The synthetic peptide may be linked to a carrier protein such as, but not limited to, keyhole limpet hemocyanin (KLH).

In a preferred embodiment, the monoclonal antibody does not specifically recognise or bind a C-extended elongated version of said C-terminus amino acid sequence or a C-truncated shortened version of said C-terminus amino acid sequence. In this regard "C-extended elongated version of said C-terminus amino acid sequence" means one or more amino acids extending beyond the C-terminus of the sequence PMKTMKGPFG-COOH (SEQ ID NO: 1). For example, if the C-terminal amino acid sequence PMKTMKGPFG-COOH (SEQ ID NO: 1) was elongated by a glycine residue then the corresponding "C-extended elongated version" would be PMKTMKGPFGG-COOH (SEQ ID NO: 2). Similarly, it is preferable that the antibody does not specifically recognise or bind a C-truncated shortened version of said C-terminus amino acid sequence. In this regard "C-truncated shortened version of said C-terminus amino acid sequence" means one or more amino acids removed from the C-terminus of the sequence PMKTMKGPFG-COOH (SEQ ID NO: 1). For example, if the C-terminal amino acid sequence PMKTMKGPFG-COOH (SEQ ID NO: 1) was shortened by one amino acid residue then the corresponding "C-truncated shortened version" would be PMKTMKGPF-COOH (SEQ ID NO: 3).

Through the use of various statistical analyses it has been found that a measured amount of binding between the monoclonal antibody (described above) and the C-terminus biomarker of 1.0 ng/mL or greater is associated with a high likelihood of ulcerative colitis or colorectal cancer. In that regard, it was found that of the total population screened (inclusive of healthy subjects, UC patients and CRC patients), at least 90% of the subjects in that population that had a said C-terminus biomarker level of ≥1.0 ng/mL suffered from UC or CRC. As such, by setting a cutoff value of 1.0 ng/mL it is possible to utilise the method of the invention to predict the likelihood of ulcerative colitis or colorectal cancer with a high level of confidence. Or, in other words, applying the statistical cutoff value to the method of the invention is particularly advantageous as it results in a standalone predictive assay i.e. it removes the need for any direct comparisons with healthy individuals and/or patients with known disease severity in order to arrive at a diagnostic conclusion. This may also be particularly advantageous when utilising the assay to evaluate patients that already have medical signs or symptoms that are generally indicative of colorectal cancer or ulcerative colitis (e.g. as determined by a physical examination and/or consultation with a medical professional) as it may act as a quick and definitive tool for corroborating the initial prognosis and thus potentially remove the need for more invasive procedures, such as an endoscopy, and expedite the commencement of a suitable treatment regimen. In the particular case of colorectal cancer an expedited conclusive diagnosis may result in the disease being detected and treated at an earlier stage, which may in turn improve overall chances of survival. The statistical cutoff value may therefore be used for assessing the risk of a patient having or developing UC or CRC. Furthermore, where the patient has Crohn's disease, it has been found that a measured amount of binding between the monoclonal antibody (described above) and the C-terminus biomarker of 1.7 ng/mL (statistical cutoff value) or greater is associated with a high likelihood of said patient having, or being likely to develop, fibrostenotic strictures. This is advantageous for much the same reasons as set out above. Thus, in a second aspect, the present invention relates to an immunoassay method for diagnosing and/or monitoring and/or assessing the likelihood of colorectal cancer or ulcerative colitis in a patient, the method comprising contacting a biofluid sample obtained from said patient with a monoclonal antibody specifically reactive with the C-terminus amino acid sequence PMKTMKGPFG (SEQ ID NO: 1) of a a C-terminus biomarker, determining the amount of binding between said antibody and collagen type XVI or fragments thereof, and correlating said amount of binding with values associated with normal healthy subjects and/or values associated with known disease severity and/or values obtained from said patient at a previous time point and/or a predetermined statistical cutoff value. Specifically, the method may be used to monitor the progression of UC or CRC in a patient, and/or the effects of therapy on a patient suffering from UC or **CRC.** For example, a first value may be obtained at a first time point prior to the commencement of therapy and a second value may be obtained at a second later time point after the commencement of therapy. A reduction in the amount of binding as measured by the method from the first time point to the second time point would be indicative of an improvement of the patient's condition, hence demonstrating the patient is responding to the therapy. Conversely, an increase in the amount of binding as measured by the method from the first time point to the second time point would be indicative of a deterioration of the patient's condition, hence demonstrating that the patient is not responding to the therapy. Accordingly, the method may be used to monitor and/or evaluate the efficacy of a novel therapeutic, such as, but not limited to, a new drug or antibody therapy.

Similarly, where the patient has Crohn's disease, the present invention relates to an immunoassay method for diagnosing the presence of fibrostenotic strictures or assessing the likelihood of development of fibrostenotic strictures in the patient with Crohn's disease, the method comprising contacting a biofluid sample obtained from said patient with an antibody specifically reactive with a C-terminus biomarker having the C-terminus amino acid sequence PMKTMKGPFG (SEQ ID NO: 1), and determining the amount of binding between said antibody and said biomarker, wherein a determined amount of binding of 1.7 ng/mL or greater is indicative of the presence of or likelihood of development of fibrostenotic strictures in said patient. Again, this method is advantageous for much the same reasons as set out above (monitoring disease progression, effects of therapy, etc.).

The detection is preferably quantitative. The fragments are preferably C-terminus fragments of Collagen type XVI.

Preferably, the antibody is specifically reactive with the C-terminus biomarker having the amino acid sequence PMKTMKGPFG (SEQ ID NO: 1). More preferably, the antibody is a monoclonal antibody. Preferably still, the antibody does not specifically recognise or bind a C-extended elongated version of said C-terminus amino acid sequence or a C-truncated shortened version of said C-terminus amino acid sequence.

For the reasons set out above, the immunoassay method of the second aspect may utilise the statistical cutoff value of 1.0 ng/mL of the C-terminus biomarker to determine the likelihood of UC or CRC.

In any of the methods of the invention described herein, the biofluid sample may be, but is not limited to, blood, urine, synovial fluid, serum or plasma.

In any of the methods of the invention described herein, the method of immunoassay may be, but is not limited to, a competition assay or a sandwich assay. The method of immunoassay may be, but is not limited to, a radioimmunoassay or an enzyme-linked immunosorbent assay.

In a third aspect the present invention relates to an assay kit comprising a monoclonal antibody specifically reactive with the amino acid sequence PMKTMKGPFG (SEQ ID NO: 1), and at least one of:
- a streptavidin coated well plate
- a biotinylated peptide Biotin-L-PMKTMKGPFG (SEQ ID NO: 4), wherein L is an optional linker
- a secondary antibody for use in a sandwich immunoassay
- a calibrator peptide comprising the sequence PMKTMKGPFG-COOH (SEQ ID NO: 1)
- an antibody biotinylation kit
- an antibody HRP labeling kit
- an antibody radiolabeling kit
- an assay visualization kit

The kit may be used for diagnosing or predicting the risk of ulcerative colitis or colorectal cancer, or for identifying patients with Crohn's disease that have or are likely to develop fibrostenotic strictures disease phenotype, preferably in conjunction with any of the methods described above.

### Definitions

As used herein the term "C-terminus" refers to the extremity of a polypeptide, i.e. at the C-terminal end of the polypeptide, and is not to be construed as meaning in the general direction thereof.

As used herein the term, the term "competitive ELISA" refers to a competitive enzyme-linked immunosorbent assay and is a technique known to the person skilled in the art.

As used herein the term "sandwich immunoassay" refers to the use of at least two antibodies for the detection of an antigen in a sample, and is a technique known to the person skilled in the art.

As used herein the term "amount of binding" refers to the quantification of binding between antibody and biomarker, which said quantification is determined by comparing the measured values of biomarker in the biofluid samples against a calibration curve, wherein the calibration curve is produced using standard samples of known concentration of the biomarker. In the specific assay disclosed herein which measures in biofluids the C-terminus biomarker having the C-terminus amino acid sequence PMKTMKGPFG (SEQ ID NO: 1), the calibration curve is produced using standard samples of known concentration of the calibration peptide PMKTMKGPFG (SEQ ID NO: 1). The values measured in the biofluid samples are compared to the calibration curve to determine the actual quantity of biomarker in the sample. The present invention utilises spectrophotometric analysis to both produce the standard curve and measure the amount of binding in the biofluid samples; in the Examples set out below the method utilises HRP and TMB to produce a measurable colour intensity which is proportional to the amount of binding and which can be read by the spectrophotometer. Of course, any other suitable analytical method could also be used.

As used herein the "statistical cutoff value" means an amount of binding that is determined statistically to be indicative of a high likelihood of UC or CRC in a patient, or indicative of a Crohn's disease (CD) patient with (or likely to develop) fibrostenotic strictures, in that a measured value of biomarker in a patient sample (preferably a serum sample) that is at or above the statistical cutoff value corresponds to at least an 80% probability, preferably at least an 85% probability, more preferably at least a 90% probability, and most preferably at least a 95% probability of the presence or likelihood of UC or CRC, or (for CD patients) the presence or likelihood of developing fibrostenotic strictures.

As used herein the term "values associated with normal healthy subjects and/or values associated with known disease severity" means standardised quantities of collagen type XVI determined by the method described supra for subjects considered to be healthy, i.e. without UC or CRC, and/or standardised quantities of collagen type XVI determined by the method described supra for subjects known to have UC or CRC of a known severity.

### Figures

**Figure 1****:** Specificity of the PRO-C16 assay (also referred to as "C16-C" in the Figures). %B/B0: B equals the OD at x nM peptide and B0 equals the OD at 0 nM peptide.

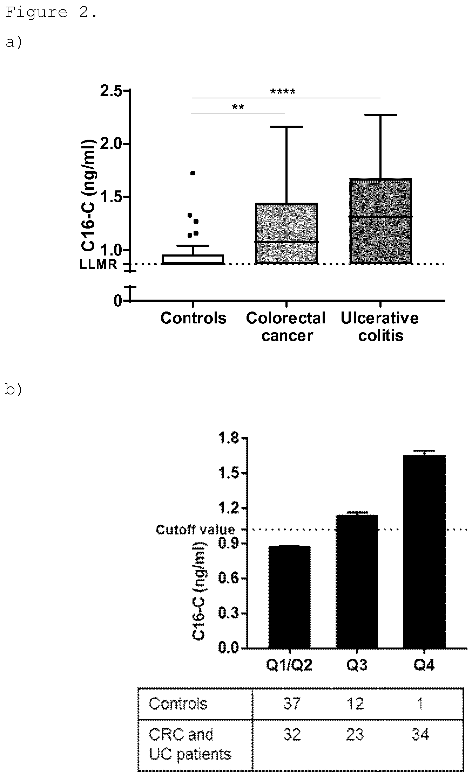

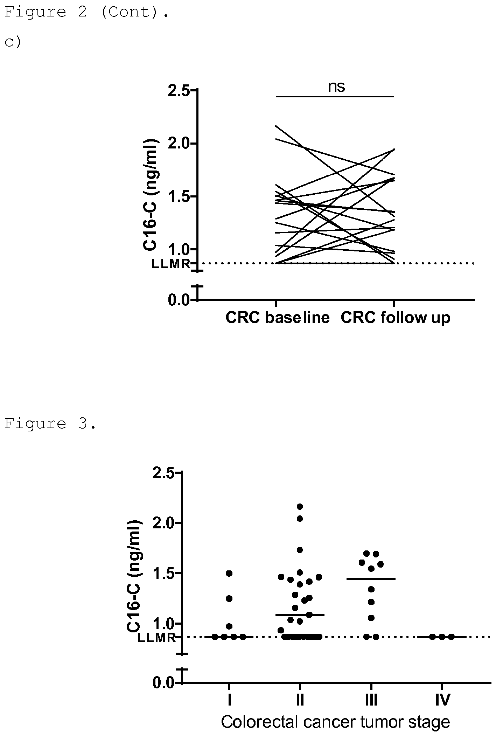

**Figure 2****:** Serum PRO-C16 levels in patients with colorectal cancer (CRC) and ulcerative colitis (UC) compared to healthy controls; (a) PRO-C16 levels in serum from controls (n=50), CRC (n=50) and UC patients (n=39). Levels below lower limit of measurement range (LLMR) are adjusted to LLMR. Results are presented as Tukey box plots. The boxes represent the 25th and 75th percentiles with a median. The whiskers represent the lowest and highest value, except outliers (•), which are higher than 1.5 times the 75th percentile. Groups were compared using Kruskal Wallis test. Asterisks indicate the following: **, p<0.01 and ****, p<0.0001; (b) Levels of PRO-C16 in serum from CRC patients, UC patients and controls divided by quartiles (Q). The number of controls, CRC and UC patients in each group are illustrated. The cutoff value (1.0 ng/mL) obtained from a ROC curve are illustrated by a dotted line; (c) PRO-C16 levels were compared in serum from CRC patients at baseline and three months after tumor resections (follow up). Statistical significant difference was determined using the paired Wilcoxon test. p>0.9999
**Figure 3****.** Evaluation of PRO-C16 in serum from colorectal cancer (CRC) patients according to tumor stage. Levels of PRO-C16 in serum from CRC patients at baseline divided into stage of disease with the median illustrated by a horizontal line. Groups were compared using Kruskal-Wallis test.
**Figure 4****.** Evaluation of PRO-C16 in serum from patients with Crohn's disease. B1: CD patients with luminal disease; B2: CD patients with fibrostenotic strictures disease phenotype; B3: CD patients with fistulizing disease phenotype.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

In the following examples, the following materials and methods were employed.

### Materials

All reagents used for the experiments were standard chemicals from Merck (Whitehouse station, NJ, USA) and Sigma Aldrich (St. Louis, MO, USA). The synthetic peptides used for antibody production and assay development were purchased from the Chinese Peptide Company (Beijing, China) (Table 1).

### Selection and overview of peptides

### Epitope Selection

The C-terminal of the α1 chain of col-16 was chosen as the target epitope and is herein referred to as "PRO-C16". The PRO-C16 amino acid sequence 1595'-PMKTMKGPFG (SEQ ID NO: 1) located at the C-terminal was used to generate an antibody specific for the C-terminal of col-16. It was additionally used to design the selection peptide (PMKTMKGPFG; SEQ ID NO: 1) (Table 1). The sequence was BLASTed for homology to other human proteins and species using the NPS@: network protein sequence analysis with the Uniprot/Swiss-Prot database (20). The amino acid sequence is unique to human col-16. A biotinylated peptide (Biotin-K-PMKTMKGPFG) was used to coat the streptavidin-coated plates applied in the ELISA. An elongated peptide (PMKTMKGPFGG; SEQ ID NO: 2), a truncated peptide (PMKTMKGPF; SEQ ID NO: 3), a nonsense peptide (VPKDLPPDTT; SEQ ID NO: 5) and a nonsense biotinylated peptide (Biotin-VPKDLPPDTT; SEQ ID NO: 6) were included to test the specificity of the antibody.

**Table 1. Synthetic peptides used for antibody production and assay development**

| **Peptide** | **Sequence** | **SEQ ID NO** |
|---|---|---|
| Selection Peptide | PMKTMKGPFG | 1 |
| Immunogenic peptide | KLH-CGG-PMKTMKGPFG | 7 |
| Biotinylated peptide | Biotin-KPMKTMKGPFG | 4 |
| Elongated peptide | PMKTMKGPFGG | 2 |
| Truncated peptide | PMKTMKGPF | 3 |
| Nonsense peptide | VPKDLPPDTT | 5 |
| Nonsense biotinylated peptide | Biotin-VPKDLPPDTT | 6 |

| | | |
|---|---|---|
| KLH, Keyhole Limpet hemocyanin | | |

### Monoclonal antibody production and clone characterization

Generation of monoclonal antibodies was carried out as previously described (21). Briefly, four to six week old Balb/C mice were immunized subcutaneously with 200 µl emulsified antigen and 50 µg immunogenic peptide (Keyhole Limpet Hemocyanin (KLH)-CGG-PMKTMKGPFG; SEQ ID NO: 7) using Freund's incomplete adjuvant (Sigma-Aldrich, St. Louis, MO, USA). The mice were immunized with two-week intervals until stable serum titer levels were reached. The mouse with the highest serum titer was selected for fusion, rested for one month, then immunized intravenously with 50 µg immunogenic peptide in 100 µl 0.9% NaCl solution. After three days, splenocytes were isolated for cell fusion. In brief, splenocytes were fused with SP2/0 myeloma cells to produce hybridoma cells, and then cloned in culture dishes using the semi-medium method (22). The clones were plated into 96-well microtiter plates and limited dilution was used to secure monoclonal growth. The supernatants were screened for reactivity against the selection peptide (PMKTMKGPFG; SEQ ID NO: 1) and native material (serum) in an indirect competitive ELISA using streptavidin-coated plates (Roche, Hvidovre, Denmark, cat. 11940279). The clones with the best reactivity were purified using protein-G-columns according to the manufacturer's instructions (GE healthcare Life Sciences, Little Chalfont, Buckinghamshire, UK). Two monoclones were tested for their reactivity towards the selection peptide (PMKTMKGPFG; SEQ ID NO: 1) and not the elongated (PMKTMKGPFGG; SEQ ID NO: 2), truncated (PMKTMKGPF; SEQ ID NO: 3) or nonsense peptide (VPKDLPPDTT; SEQ ID NO: 5). One monoclone was chosen for assay development. Optimal incubation buffer, time, temperature and optimal ratio between the biotinylated peptide and antibody was determined.

### PRO-C16 ELISA protocol

The PRO-C16 competitive ELISA procedure was as follows; a 96-well streptavidin-coated microtiter plate was coated with 100 µl of biotinylated peptide (Biotin-K-PMKTMKGPFG; SEQ ID NO: 4) dissolved in assay buffer (50mM PBS-BTB, 4g/l NaCl, pH 7.4) (final concentration of 3.1.0 ng/mL). The plate was incubated for 30 minutes at 20°C with shaking (300 rpm) and then washed five times in washing buffer (20 mM TRIS, 50 mM NaCl, pH 7.2). A volume of 20 µl of sample/control/selection peptide (PMKTMKGPFG; SEQ ID NO: 1) was added followed by immediately addition of 100 µl of monoclonal antibody diluted in assay buffer (final concentration of 62.5 ng/ml). The plate was incubated for 1 hour at 20°C with shaking followed by five washes in washing buffer. Then, 100 µl of goat antimouse HRP-conjugated IgG antibody (Thermo Scientific, Waltham, MA, USA; cat. #31437) diluted in assay buffer (final concentration of 130 ng/ml) was added to each well. The plate was incubated for 1 hour at 20°C with shaking and subsequently washed five times in washing buffer. Next, 100 µl Tetramethylbenzidine (TMB, Kem-En-Tec Diagnostics, Taastrup, Denmark) was added and incubated for 15 minutes at 20°C with shaking in the dark. To stop the reaction of TMB, 100 µl of 1% H₂SO₄ was added and the plate was analyzed in a VersaMax ELISA microplate reader at 450 nm with 650 nm as reference. A standard curve was plotted using a 4-parametric mathematical fit model and data were analyzed using the Softmax Pro v. 6.3 software.

### Technical evaluation

Antibody specificity was calculated as percentage of signal inhibition of 2-fold diluted selection peptide (PMKTMKGPFG; SEQ ID NO: 1), elongated peptide (PMKTMKGPFGG; SEQ ID NO: 2), truncated peptide (PMKTMKGPF; SEQ ID NO: 3) or nonsense peptide (VPKDLPPDTT; SEQ ID NO: 5). Lower limit of measurement range (LLMR) and upper limit of measurement range (ULMR) were calculated based on the standard curve from 10 independent runs. A 2-fold dilution of healthy serum samples from humans (n=3) were used to determine linearity and calculated as percentage recovery of the undiluted sample. Ten independent runs of seven samples that covered the detection range (LLMR-ULMR) of the PRO-C16 assay were used to calculate the intra-and-inter-assay variation. The seven samples included three human serum samples and four samples with selection peptide spiked in assay buffer. The intra-assay variation was determined as the mean coefficient of variance (CV%) within plates, and the inter-assay variation was calculated as the mean CV% between plates. Accuracy was determined from three human serum samples spiked with twofold dilutions of the selection peptide and calculated as percentage recovery of the expected concentration (serum and peptide combined). The analyte stability was determined for three healthy serum samples subjected to up to four freeze and thaw cycles. The freeze-thaw recovery was calculated with the first cycle as reference. Analyte stability was furthermore determined by incubation of three human serum samples at either 4°C or 20°C for 24 or 48 hours. Recovery was calculated with samples stored at -20°C as reference. Interference was determined by adding low/high content of biotin (1.5/4.5 ng/ml), lipid (0.75/2.5 mg/ml) or hemoglobin (1.25/2.5 mg/ml) to serum samples of known concentrations and calculated as the percentage recovery of analyte in non-spiked serum.

### Patient serum samples

Serum samples from CRC patients were collected by medical staff at Bispebjerg hospital, Copenhagen, Denmark subsequent to informed consent and approval by the Ethical Committee of the Capital Region of Denmark (Copenhagen, Denmark; approval no. H-1-2014-048) in compliance with the Helsinki Declaration. Serum samples were collected before (baseline) and three months after tumour resections (follow up) from 50 and 23 patients, respectively. Tumour staging was evaluated according to the Union for International Cancer Control classification system.

Serum samples from UC patients (n=39) were obtained from Odense University Hospital (Odense, Denmark) after informed consent. Levels of PRO-C16 in the CRC and UC patients were compared to levels in commercially available control sera from healthy donors (n=50) (Valley BioMedical, Winchester, VA, USA) which according to manufacturer's information all filed informed consent. Information associated with the included patients is shown in Table 2. According to Danish law, it is not required to get additional ethical approval when measuring biochemical markers in previously collected samples.

**Table 2. Main clinical characteristics of the study population**

| **Clinical parameter** | **Controls** | **Colorectal cancer Baseline** | **Colorectal cancer Follow up** | **Ulcerative colitis** |
|---|---|---|---|---|
| | *n=50* | *n=50* | *n=23* | *n=39* |
| Median age years (range) | 51 (19-85) | 71 (32-90) | 70 (32-83) | 32 (22-62) |
| Gender (% females) | 8% | 48% | 43.5% | 58.3% |
| Tumor stage | | | | |
| I | - | 7 | 5 | - |
| II | - | 27 | 13 | - |
| III | - | 10 | 3 | - |
| IV | - | 3 | 2 | - |
| N/A | - | 3 | | - |

### Statistical analyses

One-way analysis of variance (ANOVA) adjusted for multiple comparisons with Kruskal-Wallis test were used to compare serum levels of PRO-C16 in controls, CRC patients at baseline, and UC patients. Wilcoxon test was used to compare CRC patients at baseline and at follow up. The odds ratio and positive predictive value were generated from a specific cutoff value (1.0 ng/mL), obtained from a receiver operating characteristics (ROC) curve, and analyzed using Fisher's exact probability test and chi-square test. A p-value of p<0.05 was considered statistical significant. Statistically significant differences are marked by asterisks in figures and explained in the figure legends. Prism 7 software (Graphpad v7.01) was used for all statistical analyses.

### Specificity of the PRO-C16 assay

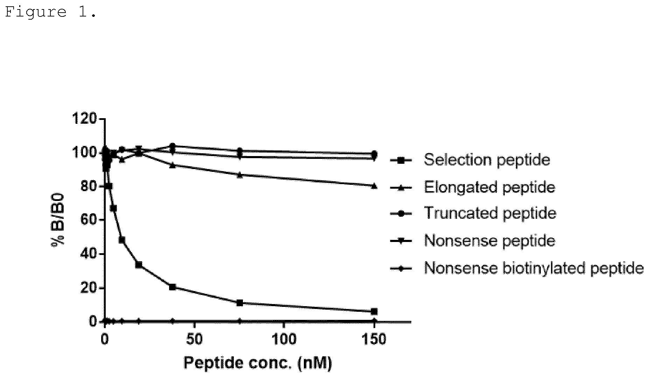

The specificity of the newly developed PRO-C16 ELISA was evaluated by investigating the inhibitory effect of different peptides. The selection peptide (PMKTMKGPFG; SEQ ID NO: 1) inhibited the signal to 6% while only a minor inhibition was detected using an elongated peptide (PMKTMKGPFGG; SEQ ID NO: 2), a truncated peptide (PMKTMKGPF; SEQ ID NO: 3) and a nonsense peptide (VPKDLPPDTT; SEQ ID NO: 5) and this only at the highest concentrations (Figure 1). No reactivity was observed toward a nonsense biotinylated peptide (Biotin-VPKDLPPDTT; SEQ ID NO: 6). Altogether, this indicates that the antibody is specific towards the C-terminal of col-16.

### Technical evaluation of the PRO-C16 assay

Several tests were included to evaluate the overall technical performance of the PRO-C16 assay (Table 3). The measurement range was determined by calculating the LLMR and ULMR, which provided a range of 0.87-95.50 ng/ml. Intra- and inter-assay variation was 10% and 15%, respectively. Native reactivity was observed in human serum. The dilution recovery in serum was 95% observed from undiluted to a 1:4 dilution. Spiking of standard peptide in human serum resulted in a mean recovery of 99%, indicating accuracy and that sample matrix do not affect assay response. The stability of the analyte was acceptable after four freeze-thaw cycles with a 103% recovery. The analyte was also recovered after prolonged storage of human serum at 4°C for 24 or 48 hours, resulting in a 106% and 95% recovery, respectively. Storage at 20°C for 24 or 48 hours, resulted in a 91% and 85% recovery, respectively. No interference was detected from either low or high levels of biotin, lipids or haemoglobin.

**Table 3. Technical validation of the PRO-C16 assay**

| **Technical validation step** | **Results** |
|---|---|
| Detection range (LLMR-ULMR) | 0.87-95.50 ng/ml |
| Intra-assay variation | 10% |
| Inter-assay variation | 15% |
| Dilution recovery in serum | 95% |
| Spiking recovery in serum | 99% |
| Freeze-thaw recovery in serum | 103% |
| Analyte stability in serum 24h, 4°C/20°C | 106% / 91% |
| Analyte stability in serum 48h, 4°C/20°C | 95% / 85% |
| Interference Biotin, low/high | 94% / 113% |
| Interference Lipid, low/high | 137% / 118% |
| Interference Hemoglobin, low/high | 97% / 100% |

| | |
|---|---|
| LLMR, Lower limit of measurement range; ULMR, Upper limit of measurement range. Percentages are reported as mean. | |

### Serum PRO-C16 levels in colorectal cancer and ulcerative colitis

To determine the biomarker potential of col-16, PRO-C16 levels were measured in serum obtained from patients with CRC and UC and compared to healthy controls. PRO-C16 levels were significantly elevated in patients with CRC (p=0.0026) and UC (p<0.0001) compared to healthy controls (Figure 2a). The percentage of CRC and UC cases of the total tested population increased stepwise with increasing quartile (Figure 2b). Of the population with PRO-C16 levels in the upper quartile (Q4), 97% (34/35) were CRC or UC patients while 3% (1/35) were healthy controls. PRO-C16 was able to identify patients with CRC or UC with a positive predictive value of 0.9 and an odds ratio of 12 (95%CI=4.5-29.5, p<0.0001).

This indicates that PRO-C16 levels are able to separate patients with CRC and UC from healthy controls. Thus, measuring PRO-C16 in serum has biomarker potential in GI disorders in general.

When PRO-C16 levels were compared (paired) between the CRC patients before tumour resections (baseline) and three months after tumour resections (follow up), no difference was observed (p>0.999) (Figure 2c). This indicates that col-16 does not originate from the primary tumour.

As the tumour stage is an important clinical tool in CRC, the PRO-C16 levels were divided according to tumour stage (Figure 3). No significant difference was detected between the tumour stages. However, a trend was observed for elevated levels of PRO-C16 in stage II and III.

### Serum PRO-C16 levels in patients with Crohn's disease

Intestinal fibrosis is a common complication in inflammatory bowel disease (IBD), but is more prevalent in Crohn's disease where it can develop into fibrostenotic strictures.

Intestinal fibroblasts and myofibroblasts are the main effector cells for intestinal fibrosis development, and intestinal subepithelial myofibroblasts (ISEM) have been shown to produce significantly elevated levels of type XVI collagen in CD patients (11).

As such, various subgroups of patients with Crohn's disease (CD) were evaluated using the PRO-C16 assay:
- CD patients with luminal disease (B1);
- CD patients with fibrostenotic strictures disease phenotype (B2); and
- CD patients with fistulizing disease phenotype (B3).

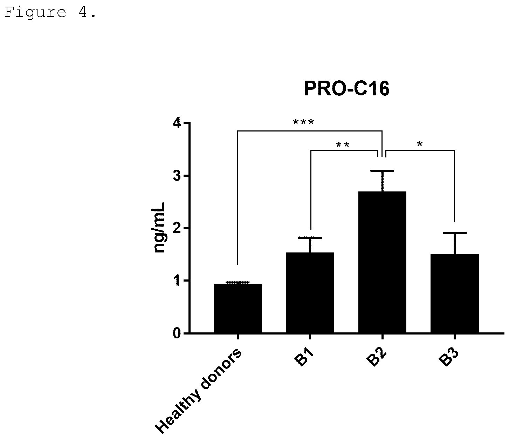

A surprisingly high (and statistically significant) level of the PRO-C16 biomarker was detected in the cohort of CD patients with fibrostenotic strictures (B2) when compared to the cohort of CD patients with luminal disease (B1), the cohort of CD patients with fistulizing disease phenotype (B3) and healthy donors (Figure 4).

These results therefore show that the PRO-C16 assay may be used to diagnose CD patients that have or are likely to develop fibrostenotic strictures. This is a significant finding, as recent reviews of the assessment of stricturing Crohn's disease (23, 24) note that there is a continuing need for non-invasive methods for evaluating strictures. Those reviews also note that currently there are no serological biomarkers that reliably predict the risk of developing intestinal strictures or identify early stages of fibrosis prior to clinical symptoms; none of the candidate biomarkers of intestinal fibrosis have been proven to be strictly specific for fibrostenosis. Accordingly, there is a continuing need for such specific serological biomarkers for non-invasive evaluation of CD patients with (or likely to develop) fibrostenotic strictures disease phenotype.

Thus, the statistically significant increase in levels of the PRO-C16 biomarker as identified in the B2 cohort indicates that the PRO-C16 assay may be used to reliably identify CD patients with (or likely to develop) fibrostenotic strictures. In that regard, for a sample obtained from a CD patient, a measured PRO-C16 value of at least 1.7 ng/mL (statistical cutoff value), and preferably at least 2.0 ng/mL, is considered to be indicative of a CD patient with (or likely to develop) fibrostenotic strictures.

### Conclusions

A robust competitive ELISA that enables non-invasive measurement of col-16 (PRO-C16) has been developed and validated. Using the herein described PRO-C16 assay, significantly elevated levels of PRO-C16 in serum from patients with CRC and UC were observed when compared with healthy controls. To our knowledge, this is the first study to show a link between Col-16 and UC or CRC, and we predict that there may be pathological links between Col-16 and other diseases, such as melanoma. Additionally, the PRO-C16 ELISA shows specificity for CD patients with (or likely to develop) fibrostenotic strictures disease phenotype, which is a significant improvement over the current serological biomarkers directed to this purpose.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. No acknowledgement of any prior published document herein should be taken to be an admission or representation that the teaching thereof was common general knowledge in Australia or elsewhere at the date hereof.

### References

1. Kehlet SN, Sanz-Pamplona R, Brix S, Leeming DJ, Karsdal MA, Moreno V. Excessive collagen turnover products are released during colorectal cancer progression and elevated in serum from metastatic colorectal cancer patients. Sci Rep [Internet]. 2016;6(1):30599. Available from: http://www.nature.com/articles/srep30599
2. Lawrance IC, Maxwell L, Doe W. Inflammation location, but not type, determines the increase in TGF-beta1 and IGF-1 expression and collagen deposition in IBD intestine. Inflamm Bowel Dis [Internet]. 2001;7(1):16-26. Available from: http://www.ncbi.nlm.nih.gov/pubmed/11233656
3. Provenzano PP, Eliceiri KW, Campbell JM, Inman DR, White JG, Keely PJ. Collagen reorganization at the tumor-stromal interface facilitates local invasion. BMC Med [Internet]. 2006;4(1):38. Available from: http://bmcmedicine.biomedcentral.com/articles/10.1186/1741-7015-4-38
4. Egeblad M, Rasch MG, Weaver VM. Dynamic interplay between the collagen scaffold and tumor evolution. Vol. 22, Current Opinion in Cell Biology. 2010. p. 697-706.
5. Iijima J, Konno K, Itano N. Inflammatory alterations of the extracellular matrix in the tumor microenvironment. Vol. 3, Cancers. 2011. p. 3189-205.
6. van Kempen LCL, de Visser KE, Coussens LM. Inflammation, proteases and cancer. Eur J Cancer. 2006;42(6):728-34.
7. Ravi A, Garg P, Sitaraman S V. Matrix metalloproteinases in inflammatory bowel disease: Boon or a bane? Vol. 13, Inflammatory Bowel Diseases. 2007. p. 97-107.
8. Shimshoni E, Yablecovitch D, Baram L, Dotan I, Sagi I. ECM remodelling in IBD: innocent bystander or partner in crime? The emerging role of extracellular molecular events in sustaining intestinal inflammation. Gut [Internet]. 2015;64(3):367-72. Available from: http://gut.bmj.com/lookup/doi/10.1136/gutjnl-2014-308048
9. Mortensen JH øg, Godskesen LE lbjerg, Jensen MD am, Van Haaften WT obias, Klinge LG abriels, Olinga P, et al. Fragments of Citrullinated and MMP-degraded Vimentin and MMP-degraded Type III Collagen Are Novel Serological Biomarkers to Differentiate Crohn's Disease from Ulcerative Colitis. J Crohns Colitis. 2015;9(10):863-72.
10. Rogler G. Chronic ulcerative colitis and colorectal cancer. Vol. 345, Cancer Letters. 2014. p. 235-41.
11. Ratzinger S, Eble JA, Pasoldt A, Opolka A, Rogler G, Grifka J, et al. Collagen XVI induces formation of focal contacts on intestinal myofibroblasts isolated from the normal and inflamed intestinal tract. Matrix Biol. 2010;29(3):177-93.
12. Grässel S, Unsöld C, Schäcke H, Bruckner-Tuderman L, Bruckner P. Collagen XVI is expressed by human dermal fibroblasts and keratinocytes and is associated with the microfibrillar apparatus in the upper papillary dermis. Matrix Biol. 1999;18(3):309-17.
13. Kassner A, Hansen U, Miosge N, Reinhardt DP, Aigner T, Bruckner-Tuderman L, et al. Discrete integration of collagen XVI into tissue-specific collagen fibrils or beaded microfibrils. Matrix Biol. 2003;22(2):131-43.
14. Grässel S, Bauer RJ. Collagen XVI in health and disease. Vol. 32, Matrix Biology. 2013. p. 64-73.
15. Eble JA, Kassner A, Niland S, Mörgelin M, Grifka J, Grässel S. Collagen XVI harbors an integrin α1β1 recognition site in its C-terminal domains. J Biol Chem. 2006;281(35):25745-56.
16. Bedal KB, Gr??ssel S, Oefner PJ, Reinders J, Reichert TE, Bauer R. Collagen XVI induces expression of MMP9 via modulation of AP-1 transcription factors and facilitates invasion of oral squamous cell carcinoma. PLoS One. 2014;9(1).
17. Ratzinger S, Grässel S, Dowejko A, Reichert TE, Bauer RJ. Induction of type XVI collagen expression facilitates proliferation of oral cancer cells. Matrix Biol. 2011;30(2):118-25.
18. Bauer R, Ratzinger S, Wales L, Bosserhoff A, Senner V, Grifka J, et al. Inhibition of collagen XVI expression reduces glioma cell invasiveness. Cell Physiol Biochem. 2011;27(3-4):217-26.
19. Senner V, Ratzinger S, Mertsch S, Grässel S, Paulus W. Collagen XVI expression is upregulated in glioblastomas and promotes tumor cell adhesion. FEBS Lett. 2008;582(23-24):3293-300.
20. Combet C, Blanchet C, Geourjon C, Deléage G. NPS@: network protein sequence analysis. Trends Biochem Sci. 2000 Mar;25(3):147-50.
21. Nielsen MJ, Nedergaard AF, Sun S, Veidal SS, Larsen L, Zheng Q, et al. The neo-epitope specific PRO-C3 ELISA measures true formation of type III collagen associated with liver and muscle parameters. Am J Transl Res. 2013;5(3):303-15.
22. Gefter ML, Margulies DH, Scharff MD. A simple method for polyethylene glycol-promoted hybridization of mouse myeloma cells. Somatic Cell Genet. 1977;3(2):231-6.
23. Giuffrida P, Pinzani M, Corazza GR, Sabatino A, Biomarkers of intestinal fibrosis - one step towards clinical trials for stricturing inflammatory bowel disease. United European Gastroenterol J. 2016 Aug; 4(4): 523-530.
24. Bettenworth D, Nowacki TM, Cordes D, Buerke B, Lenze F, Assessment of stricturing Crohn's disease: Current clinical practice and future avenues. World J Gastroenterol. 2016 Jan 21; 22(3): 1008-1016.
25. Mortensen J Høg, Manon-Jensen T, Dam Jensenz M, Haggiunda P, Klinge L G, Kjeidsen J, Krag A, Karsdal MA, Bay-Jensen A, Ulcerative colitis, Crohn's disease, and irritable bowel syndrome have different profiles of extracellular matrix turnover, which also reflects disease activity in Crohn's disease. PloS One. 2017 Oct 13; 12(10): e0185855.
26. Graham MF and Diegelmann RF Collagent content and Types in Intestinal strictures of Crohn's disease. Gastroenterology 1988;94:257-65.

## Claims

1. A method of detecting collagen type XVI or fragments thereof in a human biofluid sample, said method comprising:
contacting a biofluid sample obtained from a human patient with a monoclonal antibody specifically reactive with the C-terminus amino acid sequence PMKTMKGPFG (SEQ ID NO: 1)of a C-terminus biomarker and detecting binding between the biomarker and the antibody, preferably wherein the detection is quantitative.

2. A method as claimed in claim 1, wherein the monoclonal antibody is raised against a synthetic peptide having the amino acid sequence PMKTMKGPFG (SEQ ID NO: 1).

3. A method as claimed in claim 1 or claim 2, wherein the monoclonal antibody does not specifically recognise or bind a C-extended elongated version of said C-terminus amino acid sequence or a C-truncated shortened version of said C-terminus amino acid sequence.

4. A method as claimed in any preceding claim, wherein a measured amount of binding between the monoclonal antibody and the C-terminus biomarker of 1.0 ng/mL or greater is indicative of said human patient having or being likely to develop ulcerative colitis or colorectal cancer.

5. A method as claimed in any preceding claim, wherein the human patient has medical signs or symptoms indicative of colorectal cancer or ulcerative colitis.

6. A method as claimed in any preceding claim, wherein the human patient is a patient with Crohn's disease, and wherein a measured amount of binding between the monoclonal antibody and the C-terminus biomarker of 1.7 ng/mL or greater is indicative of said patient having or being likely to develop fibrostenotic strictures.

7. An assay kit comprising a monoclonal antibody specifically reactive with the amino acid sequence PMKTMKGPFG (SEQ ID NO: 1), and at least one of:
- a streptavidin coated well plate
- a biotinylated peptide Biotin-L-PMKTMKGPFG (SEQ ID NO: 4), wherein L is an optional linker
- a secondary antibody for use in a sandwich immunoassay
- a calibrator peptide comprising the sequence PMKTMKGPFG
- an antibody biotinylation kit
- an antibody HRP labeling kit
- an antibody radiolabeling kit
- an assay visualization kit

8. An assay kit as claimed in claim 7, wherein the monoclonal antibody is raised against a synthetic peptide having the amino acid sequence PMKTMKGPFG (SEQ ID NO: 1).

9. An assay kit as claimed in claims 7 or 8, wherein the kit is for diagnosing ulcerative colitis or colorectal cancer, or for identifying patients with Crohn's disease that have or are likely to develop fibrostenotic strictures.

10. An immunoassay method for diagnosing and/or monitoring and/or assessing the likelihood of colorectal cancer or ulcerative colitis in a patient, the method comprising contacting a biofluid sample obtained from said patient with a monoclonal antibody specifically reactive with the C-terminus amino acid sequence PMKTMKGPFG (SEQ ID NO: 1)of a C-terminus biomarker, determining the amount of binding between said antibody and collagen type XVI or fragments thereof, and correlating said amount of binding with values associated with normal healthy subjects and/or values associated with known disease severity and/or values obtained from said patient at a previous time point and/or a predetermined statistical cutoff value, preferably wherein the detection is quantitative.

11. A method as claimed in claim 10 , wherein the antibody does not specifically recognise or bind a C-extended elongated version of said C-terminus amino acid sequence or a C-truncated shortened version of said C-terminus amino acid sequence.

12. A method as claimed in claim 10 or claim 11, wherein the statistical cutoff value for the amount of binding between the monoclonal antibody and the C-terminus biomarker is at least 1.0 ng/mL.

13. An immunoassay method for diagnosing the presence of fibrostenotic strictures or assessing the likelihood of development of fibrostenotic strictures in a patient with Crohn's disease, the method comprising contacting a biofluid sample obtained from said patient with a monoclonal antibody specifically reactive with the C-terminus amino acid sequence PMKTMKGPFG (SEQ ID NO: 1) of a C-terminus biomarker and determining the amount of binding between said monoclonal antibody and said biomarker, wherein a determined amount of binding of 1.7 ng/mL or greater is indicative of the presence of or likelihood of development of fibrostenotic strictures in said patient.

14. A method as claimed in claim 13, wherein the monoclonal antibody does not specifically recognise or bind a C-extended elongated version of said C-terminus amino acid sequence or a C-truncated shortened version of said **C-**terminus amino acid sequence.

15. A method as claimed in claims 10 to 14, wherein the biofluid sample is blood, urine, synovial fluid, serum or plasma.

## Patentansprüche

1. Verfahren zum Nachweisen von Kollagen Typ XVI oder Fragmenten davon in einer menschlichen Biofluidprobe, wobei das Verfahren umfasst:
Inkontaktbringen einer von einem menschlichen Patienten erhaltenen Biofluidprobe mit einem monoklonalen Antikörper, der spezifisch mit der C-terminalen Aminosäuresequenz PMKTMKGPFG (SEQ ID NO: 1) eines C-terminalen Biomarkers reagiert, und Nachweisen der Bindung zwischen dem Biomarker und dem Antikörper, wobei der Nachweis bevorzugt quantitativ ist.

2. Verfahren nach Anspruch 1, wobei der monoklonale Antikörper gegen ein synthetisches Peptid mit der Aminosäuresequenz PMKTMKGPFG (SEQ ID NO: 1) gezüchtet wird.

3. Verfahren nach Anspruch 1 oder 2, wobei der monoklonale Antikörper eine C-verlängerte, längere Version der C-terminalen Aminosäuresequenz oder eine C-verkürzte, kürzere Version der C-terminalen Aminosäuresequenz nicht spezifisch erkennt oder bindet.

4. Verfahren nach einem der vorhergehenden Ansprüche, wobei eine gemessene Menge der Bindung zwischen dem monoklonalen Antikörper und dem C-terminalen Biomarker von 1,0 ng/ml oder mehr darauf hinweist, dass der menschliche Patient Colitis ulcerosa oder Darmkrebs hat oder wahrscheinlich entwickeln wird.

5. Verfahren nach einem der vorhergehenden Ansprüche, wobei der menschliche Patient medizinische Anzeichen oder Symptome aufweist, die auf Darmkrebs oder Colitis ulcerosa hindeuten.

6. Verfahren nach einem der vorhergehenden Ansprüche, wobei der menschliche Patient ein Patient mit Morbus Crohn ist und wobei eine gemessene Menge der Bindung zwischen dem monoklonalen Antikörper und dem C-terminalen Biomarker von 1,7 ng/ml oder mehr ein Hinweis darauf ist, dass der Patient fibrostenotische Strikturen hat oder wahrscheinlich entwickeln wird.

7. Assay-Kit, umfassend einen monoklonalen Antikörper, der spezifisch mit der Aminosäuresequenz PMKTMKGPFG (SEQ ID NO: 1) reagiert, und mindestens eines von:
- einer mit Streptavidin beschichteten Well-Platte
- einem biotinylierten Peptid Biotin-L-PMKTMKGPFG (SEQ ID NO: 4), wobei L ein optionaler Linker ist
- einen sekundären Antikörper zur Verwendung in einem Sandwich-Immunassay
- ein Kalibrierungspeptid, umfassend die Sequenz PMKTMKGPFG
- ein Antikörper-Biotinylierungs-Kit
- ein Antikörper-HRP-Markierungs-Kit
- ein Antikörper-Radiomarkierungs-Kit
- ein Assay-Visualisierungs-Kit

8. Assay-Kit nach Anspruch 7, wobei der monoklonale Antikörper gegen ein synthetisches Peptid mit der Aminosäuresequenz PMKTMKGPFG (SEQ ID NO: 1) gezüchtet wird.

9. Assay-Kit nach Anspruch 7 oder 8, wobei das Kit zur Diagnose von Colitis ulcerosa oder Darmkrebs oder zur Identifizierung von Patienten mit Morbus Crohn, die fibrostenotische Strikturen haben oder wahrscheinlich entwickeln werden, dient.

10. Immunassay-Verfahren zum Diagnostizieren und/oder Überwachen und/oder Bewerten der Wahrscheinlichkeit von Darmkrebs oder Colitis ulcerosa bei einem Patienten, wobei das Verfahren das Inkontaktbringen einer von dem Patienten erhaltenen Bioflüssigkeitsprobe mit einem monoklonalen Antikörper, der spezifisch mit der C-terminalen Aminosäuresequenz PMKTMKGPFG (SEQ ID NO: 1) eines C-terminalen Biomarkers reagiert, das Bestimmen der Menge der Bindung zwischen dem Antikörper und Kollagen Typ XVI oder Fragmenten davon und das Korrelieren der Menge der Bindung mit Werten, die normalen gesunden Subjekten zugeordnet sind, und/oder mit Werten, die einer bekannter Krankheitsschwere zugeordnet sind, und/oder mit Werten, die von dem Patienten zu einem früheren Zeitpunkt erhalten wurden, und/oder mit einem vorbestimmten statistischen Cutoff-Wert umfasst, wobei bevorzugt der Nachweis quantitativ erfolgt.

11. Verfahren nach Anspruch 10, wobei der Antikörper eine C-verlängerte, längere Version der C-terminalen Aminosäuresequenz oder eine C-verkürzte, kürzere Version der C-terminalen Aminosäuresequenz nicht spezifisch erkennt oder bindet.

12. Verfahren nach Anspruch 10 oder 11, wobei der statistische Cutoff-Wert für die Menge der Bindung zwischen dem monoklonalen Antikörper und dem C-terminalen Biomarker mindestens 1,0 ng/ml beträgt.

13. Immunassay-Verfahren zum Diagnostizieren des Vorhandenseins von fibrostenotischen Strikturen oder zum Bewerten der Wahrscheinlichkeit der Entwicklung von fibrostenotischen Strikturen bei einem Patienten mit Morbus Crohn, wobei das Verfahren das Inkontaktbringen einer von dem Patienten erhaltenen Bioflüssigkeitsprobe mit einem monoklonalen Antikörper, der spezifisch mit der C-terminalen Aminosäuresequenz PMKTMKGPFG (SEQ ID NO: 1) eines C-terminalen Biomarkers reagiert, und das Bestimmen der Menge der Bindung zwischen dem monoklonalen Antikörper und dem Biomarker umfasst, wobei eine bestimmte Menge der Bindung von 1,7 ng/ml oder mehr auf das Vorhandensein oder die Wahrscheinlichkeit der Entwicklung von fibrostenotischen Strikturen bei dem Patienten hinweist.

14. Verfahren nach Anspruch 13, wobei der monoklonale Antikörper eine C-verlängerte, längere Version der C-terminalen Aminosäuresequenz oder eine C-verkürzte, kürzere Version der C-terminalen Aminosäuresequenz nicht spezifisch erkennt oder bindet.

15. Verfahren nach einem der Ansprüche 10 bis 14, wobei die Bioflüssigkeitsprobe Blut, Urin, Synovialflüssigkeit, Serum oder Plasma ist.

## Revendications

1. Procédé de détection de collagène de type XVI ou de ses fragments dans un échantillon de fluide biologique humain, ledit procédé comprenant :
la mise en contact d'un échantillon de fluide biologique obtenu à partir d'un patient humain avec un anticorps monoclonal spécifiquement réactif avec la séquence d'acides aminés C-terminale PMKTMKGPFG (SEQ ID N° : 1) d'un biomarqueur C-terminal et la détection de la liaison entre le biomarqueur et l'anticorps, de préférence la détection étant quantitative.

2. Procédé selon la revendication 1, dans lequel l'anticorps monoclonal est dirigé contre un peptide synthétique présentant la séquence d'acides aminés PMKTMKGPFG (SEQ ID N° : 1).

3. Procédé selon la revendication 1 ou la revendication 2, dans lequel l'anticorps monoclonal ne reconnaît pas spécifiquement ni ne se lie spécifiquement à une version allongée, étendue à l'extrémité C, de ladite séquence d'acides aminés C-terminale ou une version raccourcie, tronquée à l'extrémité C, de ladite séquence d'acides aminés C-terminale.

4. Procédé selon une quelconque revendication précédente, dans lequel une quantité mesurée de liaison entre l'anticorps monoclonal et le biomarqueur C-terminal égale ou supérieure à 1,0 ng/ml indique que ledit patient humain est atteint de, ou est susceptible de développer, une colite ulcéreuse ou un cancer colorectal.

5. Procédé selon une quelconque revendication précédente, dans lequel le patient humain présente des signes ou symptômes médicaux indiquant un cancer colorectal ou une colite ulcéreuse.

6. Procédé selon une quelconque revendication précédente, dans lequel le patient humain est un patient atteint de la maladie de Crohn, et dans lequel une quantité mesurée de liaison entre l'anticorps monoclonal et le biomarqueur C-terminal égale ou supérieure à 1,7 ng/ml indique que ledit patient humain est atteint de, ou est susceptible de développer des, rétrécissements fibrosténotiques.

7. Trousse de dosage comprenant un anticorps monoclonal spécifiquement réactif avec la séquence d'acides aminés PMKTMKGPFG (SEQ ID N° : 1), et au moins l'un parmi :
- une plaque à puits revêtue de streptavidine
- un peptide biotinylé Biotine-L-PMKTMKGPFG (SEQ ID N° : 4), où L est un lieur facultatif
- un anticorps secondaire pour une utilisation dans un dosage de type sandwich
- un peptide étalon comprenant la séquence PMKTMKGPFG
- un kit de biotinylation d'anticorps
- un kit de marquage HRP d'anticorps
- un kit de radiomarquage d'anticorps
- un kit de visualisation de dosage.

8. Trousse de dosage selon la revendication 7, dans laquelle l'anticorps monoclonal est dirigé contre un peptide synthétique présentant la séquence d'acides aminés PMKTMKGPFG

9. Trousse de dosage selon les revendications 7 ou 8, la trousse étant destinée au diagnostic de la colite ulcéreuse ou du cancer colorectal, ou à l'identification de patients atteints de la maladie de Crohn qui sont atteints de, ou sont susceptibles de développer des, rétrécissements fibrosténotiques.

10. Procédé de dosage immunologique pour diagnostiquer et/ou surveiller et/ou évaluer la probabilité d'un cancer colorectal ou d'une colite ulcéreuse chez un patient, le procédé comprenant la mise en contact d'un échantillon de fluide biologique obtenu à partir dudit patient avec un anticorps monoclonal spécifiquement réactif avec la séquence d'acides aminés C-terminale PMKTMKGPFG (SEQ ID N° : 1) d'un biomarqueur C-terminal, la détermination de la quantité de liaison entre ledit anticorps et le collagène de type XVI ou ses fragments, et la corrélation de ladite quantité de liaison avec des valeurs associées à des sujets sains normaux et/ou des valeurs associées à une gravité connue de maladie et/ou des valeurs obtenues à partir dudit patient à un moment antérieur et/ou une valeur seuil statistique prédéterminée, de préférence dans lequel la détection est quantitative.

11. Procédé selon la revendication 10, dans lequel l'anticorps monoclonal ne reconnaît pas spécifiquement ni ne se lie spécifiquement à une version allongée, étendue à l'extrémité C, de ladite séquence d'acides aminés C-terminale ou une version raccourcie, tronquée à l'extrémité C, de ladite séquence d'acides aminés C-terminale.

12. Procédé selon la revendication 10 ou la revendication 11, dans lequel la valeur seuil statistique pour la quantité de liaison entre l'anticorps monoclonal et le biomarqueur C-terminal est d'au moins 1,0 ng/ml.

13. Procédé de dosage immunologique pour diagnostiquer la présence de rétrécissements fibrosténotiques ou évaluer la probabilité de développement de rétrécissements fibrosténotiques chez un patient atteint de la maladie de Crohn, le procédé comprenant la mise en contact d'un échantillon de fluide biologique obtenu à partir dudit patient avec un anticorps monoclonal spécifiquement réactif avec la séquence d'acides aminés C-terminale PMKTMKGPFG (SEQ ID N° : 1) d'un biomarqueur C-terminal et la détermination de la quantité de liaison entre ledit anticorps monoclonal et ledit biomarqueur, une quantité déterminée de liaison égale ou supérieure à 1,7 ng/ml étant indicative de la présence ou de la probabilité de développement de rétrécissements fibrosténotiques chez ledit patient.

14. Procédé selon la revendication 13, dans lequel l'anticorps monoclonal ne reconnaît pas spécifiquement ni ne se lie spécifiquement à une version allongée, étendue à l'extrémité C, de ladite séquence d'acides aminés C-terminale ou une version raccourcie, tronquée à l'extrémité C, de ladite séquence d'acides aminés C-terminale.

15. Procédé selon les revendications 10 à 14, dans lequel l'échantillon de fluide biologique est du sang, de l'urine, du liquide synovial, du sérum ou du plasma.
